(19) **Europäisches Patentamt / European Patent Office / Office européen des brevets**

(11) **EP 1 727 929 B1**

(12) **EUROPEAN PATENT SPECIFICATION**

(45) Date of publication and mention
of the grant of the patent:
**21.01.2015 Bulletin 2015/04**

(21) Application number: **05729311.0**

(22) Date of filing: **23.03.2005**

(51) Int Cl.:
***D04H 3/00*** *(2012.01)*

(86) International application number:
**PCT/US2005/009960**

(87) International publication number:
**WO 2005/095700 (13.10.2005 Gazette 2005/41)**

(54) **ABSORBENT ARTICLE WITH IMPROVED OPACITY**

SAUGKÖRPER MIT VERBESSERTER OPAZITÄT

ARTICLE ABSORBANT A OPACITE AMELIOREE

(84) Designated Contracting States:
**AT BE BG CH CY CZ DE DK EE ES FI FR GB GR HU IE IS IT LI LT LU MC NL PL PT RO SE SI SK TR**

(30) Priority: **23.03.2004 US 555502 P**

(43) Date of publication of application:
**06.12.2006 Bulletin 2006/49**

(73) Proprietor: **The Procter & Gamble Company Cincinnati, OH 45202 (US)**

(72) Inventors:
- **YOUNG, Terrill, Alan
  Cincinnati, OH 45251 (US)**
- **ISELE, Olaf, Erik, Alexander
  West Chester, OH 45069 (US)**
- **MILBRADA, Edward, John
  West Chester, OH 45069 (US)**

- **HERRON, Carlisle, Mitchell
  Cincinnati, OH 45236 (US)**

(74) Representative: **McGregor, Judit Ester et al
Procter & Gamble Service GmbH
IP Department
Frankfurter Strasse 145
61476 Kronberg im Taunus (DE)**

(56) References cited:
**WO-A-99/47088        WO-A-03/016606
US-A- 5 492 751        US-A1- 2001 004 574
US-A1- 2002 052 585**

- **PATENT ABSTRACTS OF JAPAN vol. 2000, no. 18, 5 June 2001 (2001-06-05) & JP 07 011569 A (SOLIS SRL), 13 January 1995 (1995-01-13)**

**Description**

FIELD OF INVENTION

The present invention relates to a disposable absorbent article containing a nonwoven material exhibiting improved opacity.

BACKGROUND OF THE INVENTION

Nonwoven laminates are useful for a wide variety of applications. Such nonwoven laminates are useful for wipers, towels, industrial garments, medical garments, medical drapes, and the like. In heavier basis weights the laminates are used in recreational applications such as tents, car covers, or industrial applications like geo-textiles. Disposable nonwoven laminates have achieved especially widespread use in hospital operating rooms for drapes, gowns, towels, footcovers, sterilization wraps, and the like. Additionally, disposable nonwoven laminates pervade a wide variety of absorbent articles.

Absorbent articles such as disposable diapers, training pants, incontinent wear and feminine hygiene products utilize nonwoven fabrics for many purposes such as liners, transfer layers, absorbent media, backings, and the like. For many such applications the barrier properties of the nonwoven play an important role such as in the leg cuffs as described in coassigned U.S. Pat. No. 4,900,317. As described therein, absorbent articles normally comprise three elements: a liquid permeable topsheet intended to be placed next to the wearer's skin; a liquid impermeable backsheet which forms, in use, the outer surface of the absorbent article; and an absorbent element interposed between the topsheet and the backsheet. The reference further describes breathable cuffs (i.e., leg cuffs, barrier cuffs, cuffs, etc.) which allow for the more or less free passage of vapor (including air and water vapor) through it while resisting the passage of liquid to a greater or lesser degree. Given the close proximity of the cuff to the skin, it is desirable that the nonwoven laminate be soft and comfortable.

Nonwoven laminates, particularly those in absorbent articles, are scrutinized for color consistency, softness, and opacity. In order to address consumer acceptance of absorbent articles, a great deal of effort has been spent to increasing the opacity of laminates. Such laminates often require the use of pigments such as titanium dioxide to achieve a consistent and consumer-friendly appearance. Opacity is advantageous in that increased opacity helps hide waste materials absorbed or contained in layers underlying the nonwoven laminate. Obviously, the ability to provide color consistency and opacity, as well as the other abovementioned benefits, at low cost is another consideration. Although nonwoven laminates having some combination of the properties desired have been available, it is desirable to create a nonwoven laminate that has the barrier protection, good skin feel, and increased opacity while minimizing cost and basis weight.

SUMMARY OF THE INVENTION

The present invention is directed to an absorbent article that includes a nonwoven laminate that can be made in extremely light weights and with improved opacity. The nonwoven laminates include at least one fine fibers layer and at least two continuous filaments layers, wherein the continuous filaments layers are comprised of continuous filaments having a distinct cross-sectional shape, as described herein. The nonwoven laminate may comprise a first nonwoven layer comprising first continuous filaments; a second nonwoven layer having a basis weight of less than 5 gsm, wherein the second layer comprises second continuous filaments; and a third nonwoven layer comprising fine fibers such that the total basis weight of the laminate is ideally not greater than about 100 gsm. The first and second continuous filaments of said nonwoven laminate have cross-sectional shapes that are distinct from one another. The second nonwoven layer is disposed between said first and third nonwoven layers. Furthermore, the nonwoven laminate may have a fourth nonwoven layer that is substantially similar to the second nonwoven layer wherein the third nonwoven layer is disposed between the second and fourth nonwoven layers.

In another embodiment, the absorbent article of the present invention includes a nonwoven laminate comprising a first nonwoven layer comprised of a mixture of continuous filaments with differing cross-sectional shapes; a second nonwoven layer having a basis weight of less than 5 gsm, said layer comprising second continuous filaments; and a third nonwoven layer comprising fine fibers. The nonwoven laminate comprises at least 5%, by weight, of first continuous filaments in the first nonwoven layer that are different in cross-sectional shape than the second continuous filaments in the second nonwoven layer. The second nonwoven layer is disposed between said first and third nonwoven layers. Furthermore, the nonwoven laminate may have a fourth nonwoven layer that is substantially similar to the second nonwoven layer wherein the third nonwoven layer is disposed between the second and fourth nonwoven layers.

BRIEF DESCRIPTION OF THE DRAWINGS

FIG. 1 is a schematic diagram of a forming machine used to make the nonwoven laminate of the present invention.
FIG. 2 is a cross-sectional view of the nonwoven laminate of the present invention in a three layer configuration.
FIG. 3 is a perspective view of the nonwoven laminate of FIG. 2 with cut-outs to show detail.
FIG. 4 is a cross-sectional view of the nonwoven laminate of the present invention in a four layer configuration.
FIG. 5 is a perspective view of the nonwoven laminate of FIG. 4 with cut-outs to show detail.
FIG. 6 is a cross-sectional view of continuous filaments.
FIG. 7 is a plan view of a preferred embodiment of an absorbent article as a diaper.

DETAILED DESCRIPTION OF THE INVENTION

As used herein, the term "continuous filament(s)" refers to a polymer strand that is not broken during the regular course of formation.

As used herein, the term "fine fiber(s)" refers to discrete polymer strands with an average diameter not to exceed about 10 $\mu$m.

As used herein, the term "absorbent article" refers to devices that absorb and contain liquid, and more specifically, refers to devices that are placed against or in proximity to the body of the wearer to absorb and contain the various exudates discharged from the body.

As used herein, the term "longitudinal" refers to the direction running parallel to the maximum linear dimension of the article and includes directions within $\pm 45°$ of the longitudinal direction.

As used herein, the terms "lateral" or "transverse" refers to the direction orthogonal to the longitudinal direction.

As used herein, the term "Z-direction" refers to the direction orthogonal to both the longitudinal and transverse directions.

As used herein, the term "x-y plane" refers to the plane congruent with the longitudinal and transverse directions.

As used herein, the term "disposable" is used to describe articles that generally are not intended to be laundered or otherwise restored or reused as an article (i.e., they are intended to be discarded after a single use and, preferably, to be recycled, composted or otherwise disposed of in an environmentally compatible manner).

As used herein, the term "disposed" is used to mean that an element(s) is formed (joined and positioned) in a particular place or position as a unitary structure with other elements or as a separate element joined to another element.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "unitary" absorbent article refers to absorbent articles which are formed of separate parts united together to form a coordinated entity so that they do not require separate manipulative parts like a separate holder and liner.

As used herein, the term "diaper" refers to an absorbent article generally worn by infants and incontinent persons about the lower torso.

As used herein, the term "impermeable" generally refers to articles and/or elements that are not penetrable by fluid through the entire Z-directional thickness of the article under pressure of 0.14 lb/in$^2$ or less. Preferably, the impermeable article or element is not penetrable by fluid under pressures of 0.5 lb/in$^2$ or less. More preferably, the impermeable article or element is not penetrable by fluid under pressures of 1.0 lb/in$^2$ or less.

As used herein, the term "laminate" generally refers to at least two nonwoven layers contacting along at least a portion of their respective planar faces with or without interfacial mixing.

The present invention is directed to improved lightweight nonwoven laminates including at least one fine fiber component layer and at least two continuous filament layers wherein the two continuous filament layers exhibit a distinct difference in cross-sectional filament shape from one another.

The first nonwoven layer comprises first continuous filaments with denier (g/ 9,000 m) in the range of from about 1.0 to about 3.5 (average filament diameter of from about 12 $\mu$m to about 23 $\mu$m) and a basis weight ideally not to exceed about 95 grams per square meter (gsm). Preferably, the basis weight does not exceed about 50 gsm and more preferably the basis weight does not exceed about 20 gsm. Advantageously for disposable personal care product applications, the first continuous filaments may have a denier in the range of from about 1.0 to about 2.8 (average filament diameter of from about 12 $\mu$m to about 21 $\mu$m) and a basis weight within the range of from about 4 gsm to about 10 gsm and even more preferably from about 4 to about 7 gsm. The first continuous filaments further have a relatively uniform cross-sectional shape. However, in some embodiments, the first nonwoven layer may comprise a mixture of continuous filaments with differing cross-sectional shapes.

The second nonwoven layer comprises second continuous filaments with a denier in the range from about 1.0 to about 3.5 (average filament diameter of from about 12 $\mu$m to about 23 $\mu$m) and a basis weight not to exceed about 5 gsm. Advantageously for disposable personal care product applications the continuous filaments may have a denier in the range from about 1.0 to about 2.8 (average diameter in the range of from about 12 $\mu$m to about 21 $\mu$m) and a basis weight not to exceed about 4 gsm. The second continuous filaments further have a relatively uniform cross-sectional shape. The cross-sectional shape of the second continuous filaments is distinct from that of the first continuous filaments. However, in some embodiments, only 5%, by weight, of the first continuous filaments cross-sectional shape need be distinct from the second continuous filaments.

The third nonwoven layer comprises fine fibers having an average diameter not to exceed about 10 $\mu$m and a basis weight ideally not to exceed about 50 gsm. Advantageously for applications in disposable personal care products, the average fine fiber diameter may be in the range of up to about 5 $\mu$m, and the fine fiber web basis weight may be in the range of from about 0.5 gsm to about 5 gsm.

Optionally, a fourth nonwoven layer comprises second continuous filaments with a denier in the range from about 1.0 to about 3.5 (average diameter in the range of from about 12 $\mu$m to about 23 $\mu$m) and a basis weight not to exceed about 5 gsm. Advantageously for disposable personal care product applications, the continuous filaments may have a denier in the range from about 1.0 to about 2.8 (average diameter in the range of from about 12 $\mu$m to about 21 $\mu$m) and a basis weight not to exceed about 4 gsm. The second continuous filaments further have a relatively uniform cross-sectional shape. The cross-sectional shape of the second continuous filaments is distinct from that of the first continuous filaments.

The layers yield a laminate with a total basis weight ideally not to exceed about 100 gsm and preferably not to exceed 55 gsm and more preferably not to exceed 25 gsm. However, additional layers may be present. Ideally, the layers may be bonded, but bonding is not required. Advantageously for disposable personal care product applications, the laminate basis weight is extremely low (preferably not to exceed about 20 gsm) and the fine fibers constitute a low proportion of the laminate in the range of about 5% to about 50% by weight. The resulting laminate has an improved combination of properties including increased opacity and luxurious feel. Preferred embodiments include spunbond continuous filaments webs and meltblown fine fibers webs as the respective continuous filament and fine fiber layers. On commercial equipment, a three layer laminate may be made by combining a first nonwoven layer comprised of first continuous filaments of 4.0 gsm, a second nonwoven layer comprised of second continuous filaments of 4.0 gsm, and a third nonwoven layer comprised of fine fibers of 3.0 gsm. A four layer laminate may be made by adding a fourth layer made of continuous filaments of 4.0 gsm.

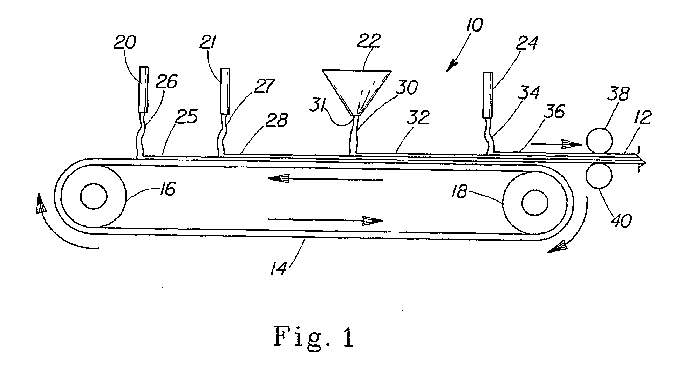

With reference to FIG. 1, a schematic diagram of a forming machine 10 used to make a preferred embodiment of the nonwoven laminate 12 is shown. The forming machine 10 is shown as having a first beam 20 for first continuous filaments 26, a second beam 21 for the second continuous filaments 27, a third beam 22 for the fine fibers 30, and an optional fourth beam 24 for the second continuous filaments 34. The forming machine 10 consists of an endless forming belt 14 wrapped around rollers 16, 18 so the belt is driven in the direction as shown by the arrows.

The first beam 20 produces the first continuous filaments 26, ideally by use of a conventional spunbond extruder with one or more spinnerets which form continuous filaments of polymer. Forming spunbond filaments and design of such a spunbond forming beam is well within the ability of those of ordinary skill in the art. Suitable thermoplastic polymers include any polymer suitable for spunbonding such as polypropylene, polyethylene, polyester, polyamide, polyimide, polyactic acid, polyhydroxyalkanoate, polyvinyl alcohol, polyacrylates, and combinations thereof. The polymer is heated to become fluid, typically at a temperature of about 100-350°C, and is extruded through the orifices in the spinneret. The extruded polymer filaments are rapidly cooled and attenuated by air streams or mechanical drafting rollers to form the desired denier filaments. The filaments resulting from the first beam are laid down onto the forming belt to create the first nonwoven layer 25. The first beam 20 may include one or more spinnerets depending upon the speed of the process or the particular polymer being used. The spinnerets of the first beam 20 have orifices with a distinct shape that imparts a cross-sectional shape to the continuous filaments. The spinnerets may be selected to yield filaments that have a trilobal shape. Furthermore, the spinnerets may be chosen so as to result in a mixture of continuous filaments with differing cross-sectional shapes. For example, the first beam 20 may comprise two spinnerets; one capable of forming continuous filaments with a substantially circular cross-sectional shape and another capable of forming continuous filaments with a trilobal cross-sectional shape. Furthermore, a spinneret could contain orifices with two or more different cross-sectional shapes. Such shapes are merely exemplars and the invention is not limited to any particular cross-sectional shapes.

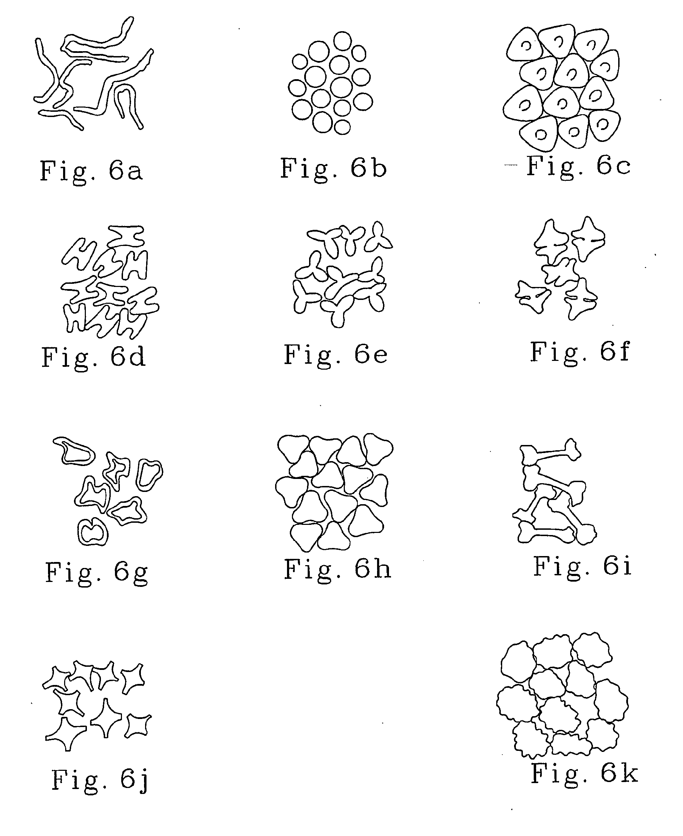

The second beam 21 produces the second continuous filaments 27, ideally by use of a conventional spunbond extruder. The extruder has a substantially similar design as the first beam 20. The second beam 21 may include one or more spinnerets depending upon the speed of the process or the particular polymer being used. The second beam 21 may involve different processing parameters than those of the first beam. For example, the polymer used in the second beam 21 may be similar or different from that used in the first beam. The temperature and attenuation may further be altered from the first beam 20. The spinnerets of the second beam 21 have orifices with a distinct shape that impart a cross-sectional shape to the continuous filaments. However, it must be noted that generally the spinnerets of the second beam 21 will yield continuous filaments 27 with a cross-sectional shape distinct from the cross-sectional shape of the first continuous filaments 26. The spinnerets may be selected to yield filaments with a cross-sectional shape including, but not limited to, those shapes selected from the group consisting of circular, oval, rectangular, square, triangular, hollow, multi-lobal, irregular, and combinations thereof. Preferably, spinnerets are selected such that the resulting filaments will have a substantially circular cross-sectional shape. FIG. 6a-k depicts several illustrative cross-sectional shapes that may be used; however, FIG. 6 is not to be read as limiting in shape selection. Additional cross-sectional shapes for the continuous filaments and methods for preparing such filaments are presented in co-assigned U.S. Patent Application serial No. 10/736,271 entitled "Hollow Fiber Fabrics" to Bond et al. and filed January 4, 2004 and U.S. Patent Application No. 11/047346 entitled "Shaped Fiber Fabrics" to Bond et al. and filed on January 28, 2005. The filaments resulting from the second beam are laid down onto first nonwoven layer 25, which is carried on the forming belt 14, to create the second nonwoven layer 28.

The third beam 22 produces fine fibers 30, preferably meltblown fibers. As known to those skilled in the art, the meltblown process results in the extrusion of a thermoplastic polymer through a die 31 containing a plurality of orifices. Ideally, the die 31 will contain from about 20 to about 40 orifices per inch of die width. As the thermoplastic polymer exits the die, high pressure fluid, usually air, attenuates and spreads the polymer stream to form fine fibers 30. The fine fibers 30 resulting from the third beam 22 are laid down onto second nonwoven layer 28, which is on the first nonwoven layer 25 carried by the forming belt 14, to create the third nonwoven layer 32. The construction and operation of the third beam 22 for forming fine fibers 30 and the third nonwoven layer 32 is considered conventional, and the design and operation are well within the ability of those of ordinary skill in the art. Such skill is demonstrated by NRL Report 4364, "Manufacture of Super-Fine Organic Fibers", by V. A. Wendt, E. L. Boon, and C. D. Fluharty; NRL Report 5265, "An Improved Device for the Formation of Super-Fine Thermoplastic Fibers", by K. D. Lawrence, R. T. Lukas, and J. A. Young; and U.S. Pat. No. 3,849,241, issued Nov. 19, 1974, to Buntin et al. Other methods for forming a nonwoven web of fine fibers are contemplated for use with the present invention.

In another preferred embodiment, the forming machine may include a fourth beam 24 to produce a fourth nonwoven layer 36 comprising second continuous filaments 34, ideally by use of a conventional spunbond extruder. The spunbond extruder consists of spinnerets which form continuous filaments of a polymers; the extruder has a substantially similar design as that of the second beam 21. The fourth beam 24 may include one or more spinnerets depending upon the speed of the process or the particular polymer being used. The fourth beam 24 may involve different processing parameters. For example, the polymer used in the fourth beam 24 may be similar or different from that used in the first beam 20. The temperature and attenuation may further be altered from the first beam 20. The spinnerets of the fourth beam 24 have orifices with a distinct shape that impart a cross-sectional shape to the continuous filaments. However, it must be noted that generally the spinnerets of the fourth beam 24 will yield continuous filaments 34 with a cross-sectional shape distinct from the cross-sectional shape of the first continuous filaments. The spinnerets may be selected to yield filaments with cross-sectional shape including, but not limited to, those shapes selected from the group consisting of circular, oval, rectangular, square, triangular, hollow, multi-lobal, irregular, and combinations thereof. Preferably, spinnerets are selected to be identical to the second beam 21; namely, the resulting filaments will ideally have a substantially circular cross-sectional shape. FIG. 6a-k depicts several illustrative cross-sectional shapes that may be used; however, FIG. 6 is not to be read as limiting in shape selection. Additional cross-sectional shapes for the continuous filaments and methods for preparing such filaments are presented in coassigned U.S. Patent Application serial No. 10/736,271 entitled "Hollow Fiber Fabrics" to Bond et al. and filed January 4, 2004 and U.S. Patent Application No. 11/047346 entitled "Shaped Fiber Fabrics" to Bond et al. and filed on January 28, 2005. The filaments resulting from the fourth beam 24 are laid down onto third nonwoven layer 32, which is carried on the forming belt 14, to create the fourth nonwoven layer 36.

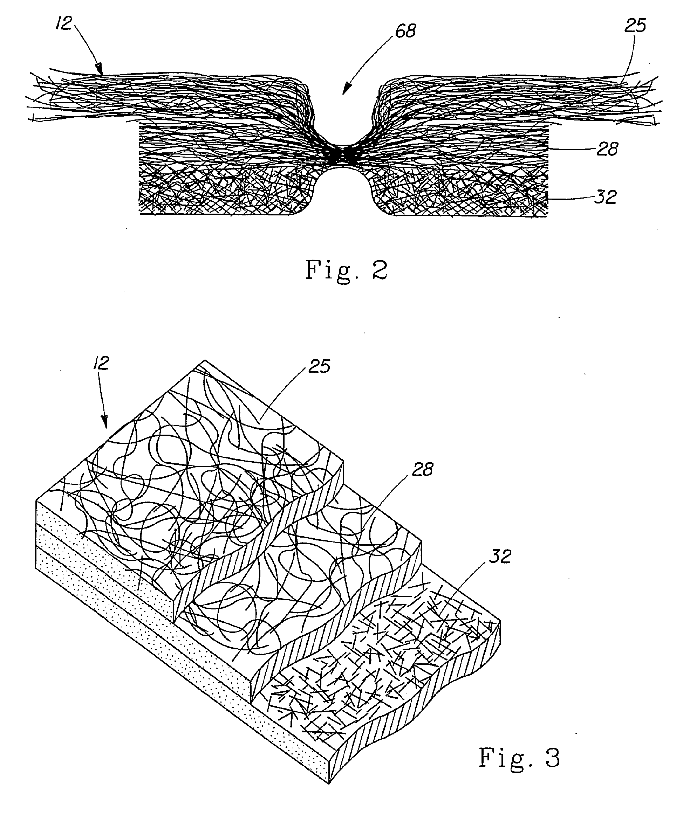

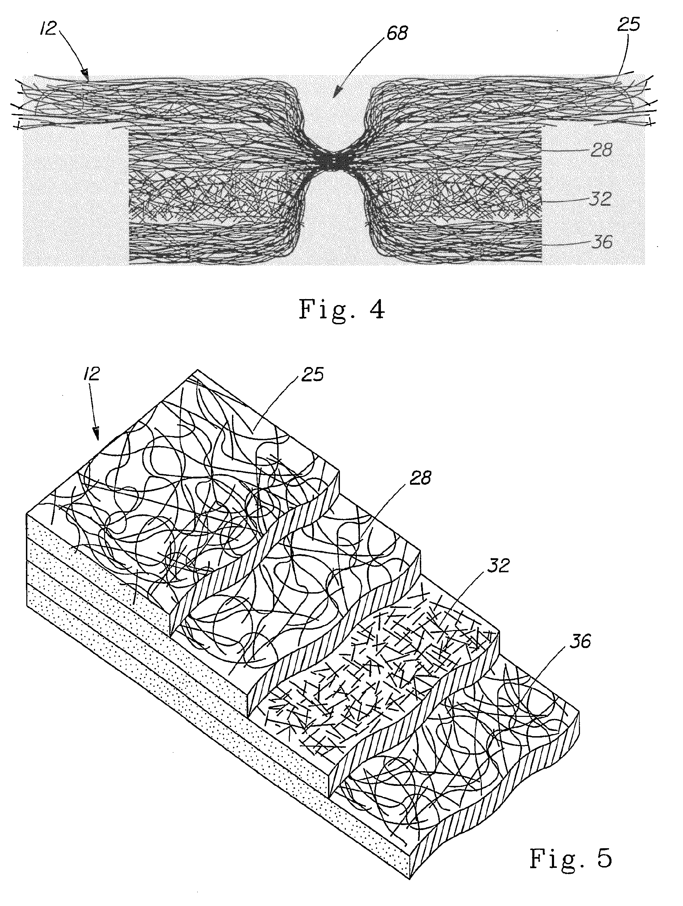

The resulting nonwoven laminate 12 may be fed through bonding rolls 38, 40. The surfaces of one or both of the bonding rolls 38, 40 may be provided with a raised pattern such as spots or grids. The bonding rolls are heated to the softening temperature of the polymer used to form the layers of the laminate 12. As the laminate 12 passes between the heated bonding rolls 38, 40, the material is embossed by the bonding rolls in accordance with the pattern on the rolls to create a pattern of discrete areas, such as 68 shown in FIGS. 2 and 4, said areas are bonded from layer to layer with respect to the particular filaments and/or fibers within each layer. Such discrete area or spot bonding is well-known in the art and can be carried out as described by means of heated rolls or by means of ultrasonic heating of the laminate 12 to produced discrete area thermally bonded filaments, fibers, and layers. In accordance with conventional practice described in Brock et al., U.S. Pat. No. 4,041,203, it is preferable for the fibers of the meltblown layer in the fabric laminate to fuse within the bond areas while the filaments of the spun-bonded layers retain their integrity in order to achieve good strength characteristics. For heavier basis weight laminates, for example, sonic bonding as described in U.S. Pat. No. 4,374,888 may be preferred. However, other bonding methods known in the art may be used. Furthermore, it is envisioned that the nonwoven laminate may be created from discrete nonwoven layers that are formed, rolled, and later laminated by methods well known in the art (including stacking the discrete layers without bonding) rather than the layers being laid by a single forming machine as presented above.

The resulting nonwoven laminate 12 may be subjected to a method for selectively aperturing the laminate. As disclosed in co-assigned U.S. Patent Nos. 5,628,097 and 5,916,661 and U.S. Patent App. Serial No. 09/909486; the nonwoven laminate may be weakened at a plurality of locations on the laminate, such as by a patterned calendar roller, to create a plurality of weakened, melt-stabilized locations. The weakened laminate is subjected to an incremental stretching means by an incremental stretching means, such as ring rolling or tentering, causing the nonwoven laminate to rupture at the plurality of weakened, melt-stabilized locations. The resulting nonwoven laminate comprises a plurality of apertures coincident with the plurality of weakened, melt-stabilized locations.

While a nonwoven laminate is described as preferably comprising three to four nonwoven layers, greater or lesser numbers of layers may be used and are clearly envisioned by this disclosure.

FIG. 2 illustrates a cross-sectional view of a preferred nonwoven laminate embodiment. FIG. 3 illustrates the same preferred nonwoven laminate in a perspective view with cut-aways to show detail. Shown is a three layer nonwoven laminate 12 created from the forming machine described above without the optional fourth beam. The nonwoven laminate comprises a first nonwoven layer 25 which itself is comprised of first continuous filaments, preferably spunbond filaments. The first nonwoven layer preferably has a basis weight from about 4 gsm to about 7 gsm. Most preferably, the basis weight of the first nonwoven layer is about 4 gsm. The first continuous filaments have cross-sectional shapes including, but not limited to, those shapes selected from the that is trilobal. The nonwoven laminate comprises a second nonwoven layer which itself is comprised of second continuous filaments, preferably spunbond filaments. The second nonwoven layer 28 preferably has a basis weight not to exceed about 5 gsm. Most preferably, the basis weight of the second nonwoven layer does not exceed about 4 gsm. The second continuous filaments have a cross-sectional shape distinct from the cross-sectional shape of the first continuous filaments. The second continuous filaments may have cross-sectional shapes including, but not limited to, those shapes selected from the group consisting of circular, oval, rectangular, square, triangular, hollow, multi-lobal, irregular, and combinations thereof. Most preferably, the cross-sectional shape is substantially circular. The nonwoven laminate comprises a third nonwoven layer 32 which itself is comprised of fine fibers, preferably meltblown fibers. The third nonwoven layer preferably has a basis weight from about 0.5 gsm to about 5.0 gsm. Most preferably, the basis weight of the third nonwoven layer is from about 1.5 gsm to about 3 gsm. The total basis weight of the three layered laminate ideally will not exceed about 100 gsm. Preferably, the total basis weight of the three layered laminate will not exceed about 55 gsm. More preferably, the total basis weight of the three layered laminate will not exceed about 25 gsm. Most preferably, the total basis weight is from about 11 gsm to about 13 gsm. The resultant laminate has the second nonwoven layer interposed between the first and third nonwoven layers. Thermal bonds 68 (not shown in FIG. 3) may be used to adhere layer to layer with respect to the particular filaments and/or fibers within the layer.

FIG. 4 illustrates a cross-sectional view of a preferred nonwoven laminate embodiment. FIG. 5 illustrates the same preferred nonwoven laminate in a perspective view with cut-aways to show detail. Shown is a four layer nonwoven laminate 12 created from the forming machine described above with the optional fourth beam. The first nonwoven layer 25, the second nonwoven layer 28, and the third nonwoven layer 32 are substantially similar to the three layer laminate as described immediately above. The nonwoven laminate comprises a fourth nonwoven layer 36 which itself is comprised of second continuous filaments, preferably spunbond filaments. The fourth nonwoven layer 36 preferably has a basis weight not to exceed about 5 gsm. Most preferably, the basis weight of the fourth nonwoven layer does not exceed about 4 gsm. The second continuous filaments have a cross-sectional shape distinct from the cross-sectional shape of the first continuous filaments. The second continuous filaments may have cross-sectional shapes including, but not limited to, those shapes selected from the group consisting of circular, oval, rectangular, square, triangular, hollow, multi-lobal, irregular, and combinations thereof. Most preferably, the cross-sectional shape is substantially circular. The total basis weight of the four layered laminate will ideally not exceed about 100 gsm. Preferably, the total basis weight of the three layered laminate will not exceed about 55 gsm. More preferably, the total basis weight of the three layered laminate will not exceed 25 gsm. In the most preferred embodiment, the four layered laminate has a basis weight from about 15 gsm to about 17 gsm. The resultant laminate yields the second nonwoven layer interposed between the first and third nonwoven layers and the third nonwoven layer interposed between the second and fourth nonwoven layers. Thermal bonds 68 (not shown in FIG. 3) may be used to adhere layer to layer with respect to the particular filaments and/or fibers within the layer.

In another preferred embodiment, the nonwoven laminate is a three layer laminate with two continuous filaments layers and one fine fibers layer, wherein the first nonwoven layer may comprise a mixture of continuous filaments with differing cross-sectional shapes. Similar to the three layer laminate presented above, the present embodiment comprises a first nonwoven layer of first continuous filaments, a second nonwoven layer of second continuous filaments, and a third nonwoven layer of fine fibers. The continuous filaments layers may comprise a mixture of filaments with differing cross-sectional shapes. Preferably, the first nonwoven layer comprises continuous filaments with differing cross-sectional shapes such that at least 5%, by weight, of the first continuous filaments are distinct from the cross-sectional shape of the second continuous filaments of the second layer. For example, the first nonwoven layer may comprise first continuous filaments of which 95%, by weight, of the filaments are substantially circular in cross-sectional shape and 5%, by weight, of the filaments are trilobal in cross-sectional shape, and the second nonwoven layer may comprise second continuous filaments which are substantially circular in cross-sectional shape. However, the first nonwoven layer may comprise first continuous filaments with differing cross-sectional shapes of other varying ratios. Furthermore, the first nonwoven layer may include more than two filaments with distinct cross-sectional shapes. Preferably, the first continuous filaments are (by weight percent) 50% trilobal and 50% circular. The first nonwoven layer preferably has a basis weight from about 4 gsm to about 7 gsm. Most preferably, the basis weight of the first nonwoven layer is about 4 gsm. The second nonwoven layer preferably has a basis weight not to exceed about 5 gsm. Most preferably, the basis weight of the second nonwoven layer does not exceed about 4 gsm. The nonwoven laminate comprises a third nonwoven layer which itself is comprised of fine fibers, preferably meltblown fibers. The third nonwoven layer preferably has a basis weight from about 0.5 gsm to about 5.0 gsm. Most preferably, the basis weight of the third nonwoven layer is about 3 gsm. The total basis weight of the three layered laminate will ideally not exceed about 100 gsm and more preferably will not exceed 55 gsm. Even more preferably, the basis weight of the three layered laminate will not exceed about 25 gsm. In the most preferred embodiment the total basis weight of the three layered laminate is from about 11 gsm to about 13 gsm. The resultant laminate has the second nonwoven layer interposed between the first and third nonwoven layers. Thermal bonds may be used to adhere layer to layer with respect to the particular filaments and or fibers within the layer.

In another preferred embodiment, the nonwoven laminate is a four layer laminate with three continuous filaments layers and one fine fibers layer, wherein at least one of the continuous filaments layers comprise a mixture of continuous filaments with differing cross-sectional shapes. The present embodiment comprises a first nonwoven layer of first continuous filaments, a second nonwoven layer of second continuous filaments, a third nonwoven layer of fine fibers, and a fourth nonwoven layer of second continuous filaments. The continuous filaments layers may comprise a mixture of filaments with differing cross-sectional shapes. In a particularly preferred embodiment, the first, second, and third nonwoven layers are similar to those presented in the immediately aforementioned embodiment. In addition to the aforementioned three layer nonwoven laminate, the nonwoven laminate comprises a fourth nonwoven layer which itself is comprised of second continuous filaments, preferably spunbond filaments. The fourth nonwoven layer preferably has a basis weight not to exceed about 5 gsm. Most preferably, the basis weight of the fourth nonwoven layer does not exceed about 4 gsm. The total basis weight of the four layered laminate will ideally not exceed about 100 gsm and more preferably will not exceed about 55 gsm. Even more preferably, the total basis weight of the four layered laminate will not exceed about 25 gsm. In the most preferred embodiment, the total basis weight of the four layered laminate is from about 15 gsm to about 17 gsm. The resultant laminate yields the second nonwoven layer interposed between the first and third nonwoven layers and the third nonwoven layer interposed between the second and fourth nonwoven layers. Thermal bonds may be used to adhere layer to layer with respect to the particular filaments and or fibers within the layer.

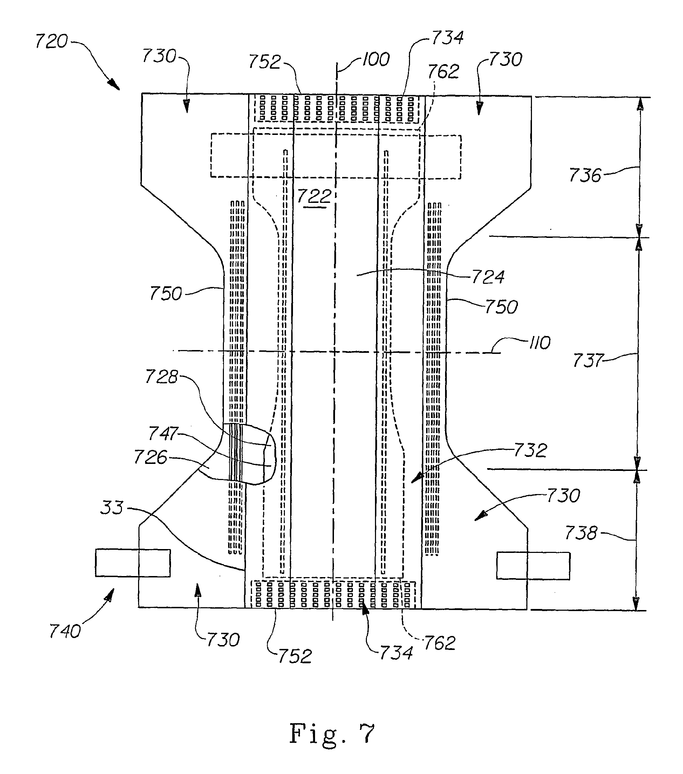

FIG. 7 is a plan view of the diaper 720 of the present invention in a flat-out state with portions of the structure being cut-away to more clearly show the construction of the diaper 720. The portion of the diaper 720 which faces the wearer is oriented towards the viewer. As shown in FIG. 7, the diaper 720 preferably comprises a liquid pervious topsheet 724; a liquid impervious backsheet 726; an absorbent core 728 which is preferably positioned between at least a portion of the topsheet 724 and the backsheet 726; side panels 730; elasticized leg cuffs 732; an elastic waist feature 734; and a fastening system generally designated 740. The diaper 720 is shown in FIG. 7 to have a first waist region 736, a second waist region 738 opposed to the first waist region 736 and a crotch region 737 located between the first waist region 736 and the second waist region 738. The periphery of the diaper 720 is defined by the outer edges of the diaper 720 in which longitudinal edges 750 run generally parallel to the longitudinal centerline 100 of the diaper 720 and end edges 752 run between the longitudinal edges 750 generally parallel to the lateral centerline 110 of the diaper 720.

The chassis 722 of the diaper 720 comprises the main body of the diaper 720. The chassis 722 comprises at least a portion of the absorbent core 728 and preferably an outer covering including the topsheet 724 and/or the backsheet 726. If the absorbent article comprises a separate holder and a liner, the chassis 722 generally comprises the holder and the liner. (For example, the holder may comprise one or more layers of material to form the outer cover of the article and the liner may comprise an absorbent assembly including a topsheet, a backsheet, and an absorbent core. In such cases, the holder and/or the liner may include a fastening element which is used to hold the liner in place throughout the time of use.) For unitary absorbent articles, the chassis 722 comprises the main structure of the diaper with other features added to form the composite diaper structure. While the topsheet 724, the backsheet 726, and the absorbent core 728 may be assembled in a variety of well known configurations, preferred diaper configurations are described generally in U.S. Pat. No. 3,860,003 entitled "Contractible Side Portions for Disposable Diaper" issued to Kenneth B. Buell on January 14, 1975; U.S. Pat. No. 5,151,092 issued to Buell on September 9, 1992; and U.S. Pat. No. 5,221,274 issued to Buell on June 22, 1993; and U.S. Pat. No. 5,554,145 entitled "Absorbent Article With Multiple Zone Structural Elastic-Like Film Web Extensible Waist Feature" issued to Roe et al. on September 10, 1996; U.S. Pat. No. 5,569,234 entitled "Disposable Pull-On Pant" issued to Buell et al. on October 29, 1996; U.S. Pat. No. 5,580,411 entitled "Zero Scrap Method For Manufacturing Side Panels For Absorbent Articles" issued to Nease et al. on December 3, 1996; and U.S. Patent No. 6,004,306 entitled "Absorbent Article With Multi-Directional Extensible Side Panels" issued to Robles et al. on December 21, 1999; each of which is incorporated herein by reference.

The backsheet 726 is generally that portion of the diaper 720 positioned adjacent garment facing surface 745 of the absorbent core 728 which prevents the exudates absorbed and contained therein from soiling articles which may contact the diaper 720, such as bedsheets and undergarments. In preferred embodiments, the backsheet 726 is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Corporation, based in Richmond, VA, and sold under the trade name CPC2 film. Exemplary breathable materials may include materials such as woven webs, nonwoven webs, composite materials such as film-coated nonwoven webs, microporous films such as manufactured by Mitsui Chemical Co., of Japan under the designation ESPOIR NO and by Tredegar Corporation of Richmond, VA and sold under the designation EXAIRE., and monolithic films such as manufactured by Clopay Corporation, Cincinnati, OH under the name HYTREL blend P18-3097. Some breathable composite materials are described in greater detail in PCT Application No. WO 95/16746 published on June 22, 1995 in the name of E. I. DuPont; U.S. Patent No. 5,938,648 issued on August 17, 1999 to LaVon et al.; U.S. Pat. No. 5,865,823 issued on February 2, 1999 in the name of Curro; and U.S. Pat. No. 5,571,096 issued to Dobrin et al. on November 5, 1996.

The backsheet 726, or any portion thereof, may be elastically extensible in one or more directions. In one embodiment, the backsheet 726 may comprise a structural elastic-like film ("SELF") web as described in more detail in U.S. Patent No. 5,518,801 entitled "Web Materials Exhibiting Elastic-Like Behavior" issued to Chappell, et al. on May 21, 1996. In alternate embodiments, the backsheet 726 may comprise elastomeric films, foams, strands, or combinations of these or other suitable materials with nonwovens or synthetic films.

The backsheet 726 may be joined to the topsheet 724, the absorbent core 728 or any other element of the diaper 720 by any attachment means known in the art. For example, the attachment means may include a uniform continuous layer of adhesive, a patterned layer of adhesive, or an array of separate lines, spirals, or spots of adhesive. One preferred attachment means comprises an open pattern network of filaments of adhesive as disclosed in U.S. Patent 4,573,986 entitled "Disposable Waste-Containment Garment", which issued to Minetola et al. on March 4, 1986. Other suitable attachment means include those illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Adhesives which have been found to be satisfactory are manufactured by H. B. Fuller Company of St. Paul, Minnesota and marketed as HL-1620 and HL-1358-XZP. Alternatively, the attachment means may comprise heat bonds, pressure bonds, ultrasonic bonds, dynamic mechanical bonds, or any other suitable attachment means or combinations of these attachment means as are known in the art.

The topsheet 724 is preferably positioned adjacent body surface 747 of the absorbent core 728 and may be joined thereto and/or to the backsheet 726 by any attachment means known in the art. Suitable attachment means are described above with respect to means for joining the backsheet 726 to other elements of the diaper 720. In one preferred embodiment of the present invention, the topsheet 724 and the backsheet 726 are joined directly to each other in some locations and are indirectly joined together in other locations by directly joining them to one or more other elements of the diaper 720.

The topsheet 724 is preferably compliant, soft-feeling, and non-irritating to the wearer's skin. Further, at least a portion of the topsheet 724 is liquid pervious, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers (e.g., polyester or polypropylene fibers), or a combination of natural and synthetic fibers. If the topsheet 724 includes fibers, the fibers may be spunbond, carded, wet-laid, meltblown, hydroentangled, or otherwise processed as is known in the art. One suitable topsheet 724 comprising a web of hydrophilically treated spunbond polypropylene is manufactured by BBA Fiberweb, Old Hickory, TN, under the designation P10 or 055SLPI09E for example.

Suitable formed film topsheets are described in U.S. Pat. No. 3,929,135 issued to Thompson on December 30, 1975; U.S. Pat. No. 4,324,246 issued to Mullane, et al. on April 13, 1982; U.S. Patent 4,342,314 issued to Radel, et al. on August 3, 1982; U.S. Pat. No. 4,463,045 issued to Ahr, et al. on July 31, 1984; and U.S. Pat. No. 5,006,394 issued to Baird on April 9, 1991. Other suitable topsheets 724 may be made in accordance with U.S. Pat. Nos. 4,609,518 and 4,629,643 issued to Curro et al. on September 2, 1986 and December 16, 1986, respectively. Such formed films are available from The Procter & Gamble Company of Cincinnati, Ohio as "DRI-WEAVE" and from Tredegar Corporation, based in Richmond, VA, as "CLIFF-T." Another suitable topsheet is disclosed in U.S. Publication 2004/0092902 A1 to Anja Hoffmann et al.

Preferably, at least a portion of the topsheet 724 is made of a hydrophobic material or is treated to be hydrophobic in order to isolate the wearer's skin from liquids contained in the absorbent core 728. If the topsheet 724 is made of a hydrophobic material, preferably at least a portion of the upper surface of the topsheet 724 is treated to be hydrophilic so that liquids will transfer through the topsheet more rapidly. The topsheet 724 can be rendered hydrophilic by treating it with a surfactant or by incorporating a surfactant into the topsheet. Suitable methods for treating the topsheet 724 with a surfactant include spraying the topsheet 724 material with the surfactant and/or immersing the material into the surfactant. A more detailed discussion of such a treatment and hydrophilicity is contained in U.S. Pat. No. 4,988,344 entitled "Absorbent Articles with Multiple Layer Absorbent Layers" issued to Reising, et al. on Jan. 29, 1991 and U.S. Pat. No. 4,988,345 entitled "Absorbent Articles with Rapid Acquiring Absorbent Cores" issued to Reising on Jan. 29, 1991. A more detailed discussion of some suitable methods for incorporating a surfactant in the topsheet 724 can be found in U.S. Statutory Invention Registration No. H1670 published on July 1, 1997 to Aziz et al. Alternatively, the topsheet 724 may include an apertured web or film which is hydrophobic. Hydrophobicity is achieved by eliminating the hydrophilizing treatment step from the production process and/or applying a hydrophobic treatment to the topsheet 724, such as a flouryl, paraffin, or silicone-based compound like Repellan ZN by Cognis Corporation or a hydrophobic lotion composition, as described below. In such embodiments, it is preferred that the apertures be large enough to allow the penetration of aqueous fluids like urine without significant resistance.

Any portion of the topsheet 724 may be coated with a lotion as is known in the art. Examples of suitable lotions include those described in U.S. Pat. Nos. 5,607,760 issued to Roe on March 4, 1997; U.S. Pat. No. 5,609,587 issued to Roe on March 11, 1997; U.S. Pat. No. 5,635,191 issued to Roe et al. on June 3, 1997; U.S. Pat. No. 5,643,588 issued to Roe et al. on July 1, 1997; and U.S. Pat. No. 5,968,025 issued to Roe et al. on October 19, 1999. The lotion may function alone or in combination with another agent as the hydrophobizing treatment described above. The topsheet 724 may also include or be treated with antibacterial agents, such as disclosed in PCT Publication No. WO 95/24173 entitled "Absorbent Articles Containing Antibacterial Agents in the Topsheet For Odor Control" and published on September 14, 1995 to Theresa Johnson. Further, the topsheet 724, the backsheet 726 or any portion of the topsheet or backsheet may be embossed and/or matte finished to provide a more cloth like appearance.

The topsheet 724 may comprise one or more apertures to ease penetration of exudates therethrough, such as urine and/or feces (solid, semi-solid, or liquid). The size of at least the primary aperture is important in achieving the desired waste encapsulation performance. If the primary aperture is too small, the waste may not pass through the aperture, either due to poor alignment of the waste source and the aperture location or due to fecal masses having a diameter greater than the aperture If the aperture is too large, the area of skin that may be contaminated by "rewet" from the article is increased. Typically, the aperture should have an area of between about 10 cm$^2$ and about 50 cm$^2$. The aperture preferably has an area of between about 15 cm$^2$ and 35 cm$^2$.

Further, the topsheet 724 may be fully or partially elasticated or may be foreshortened to provide a void space between the topsheet 724 and the core 728. Exemplary structures including elasticized or foreshortened topsheets are described in U.S. Pat. No. 4,892,536 issued to DesMarais et al. on January 9, 1990; U.S. Pat. No. 4,990,147 issued to Freeland on February 5, 1991; U.S. Pat. No. 5,037,416 issued to Allen et al. on August 6, 1991; U.S. Pat. No. 5,269,775 issued to Freeland et al. on December 14, 1993; U.S. Patent No. 6,482,191 to Roe et al.; U.S. Pat. Application No. 10/764,850 entitled "Articles with Elasticated Topsheets" filed on January 26, 2004 to Joerg Mueller et al.; and U.S. Pat. Application No. 10/703,239 entitled "Disposable Absorbent Articles With Masking Topsheet" filed on November 7, 2003 to Anja Hoffmann et al. Further, a diaper may comprise more than one topsheet as is described in U.S. Publication 2004/0092900 A1 entitled "Disposable Absorbent Article With Improved Topsheet" and filed on November 7, 2003 to Anja Hoffmann et al.

The absorbent core 728 may comprise any absorbent material which is generally compressible, conformable, non-irritating to the wearer's skin, and capable of absorbing and retaining liquids such as urine and other certain body exudates. The absorbent core 728 may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The configuration and construction of the absorbent core 728 may also be varied (e.g., the absorbent core(s) or other absorbent structure(s) may have varying caliper zones, hydrophilic gradient(s), a superabsorbent gradient(s), or lower average density and lower average basis weight acquisition zones; or may comprise one or more layers or structures). Exemplary absorbent structures for use as the absorbent core 728 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,834,735 entitled "High Density Absorbent Members Having Lower Density and Lower Basis Weight Acquisition Zones" issued to Alemany et al. on May 30, 1989; U.S. Patent 4,888,231 entitled "Absorbent Core Having A Dusting Layer" issued to Angstadt on December 19, 1989; U.S. Pat. No. 5,137,537 entitled "Absorbent Structure Containing Individualized, Polycarboxylic Acid Crosslinked Wood Pulp Cellulose Fibers" issued to Herron et al. on August 11, 1992; U.S. Patent 5,147,345 entitled "High Efficiency Absorbent Articles For Incontinence Management" issued to Young et al. on September 15, 1992; U.S. Pat. No. 5,342,338 entitled "Disposable Absorbent Article For Low-Viscosity Fecal Material" issued to Roe on August 30, 1994; U.S. Pat. No. 5,260,345 entitled "Absorbent Foam Materials For Aqueous Body Fluids and Absorbent Articles Containing Such Materials" issued to DesMarais et al. on November 9, 1993; U.S. Pat. No. 5,387,207 entitled "Thin-Until-Wet Absorbent Foam Materials For Aqueous Body Fluids And Process For Making Same" issued to Dyer et al. on February 7, 1995; and U.S. Pat. No. 5,625,222 entitled "Absorbent Foam Materials For Aqueous Fluids Made From High Internal Phase Emulsions Having Very High Water-To-Oil Ratios" issued to DesMarais et al. on July 22, 1997.

The diaper 720 may also include a sublayer disposed between the topsheet 724 and the backsheet 726. The sublayer may be any material or structure capable of accepting, storing or immobilizing bodily exudates. Thus, the sublayer may include a single material or a number of materials operatively associated with each other. Further, the sublayer may be integral with another element of the diaper 720 or may be one or more separate elements joined directly or indirectly with one or more elements of the diaper 720. Further, the sublayer may include a structure that is separate from the core 728 or may include or be part of at least a portion of the core 728.

Suitable materials for use as the sublayer may include large cell open foams, macro-porous compression resistant nonwoven highlofts, large size particulate forms of open and closed cell foams (macro and/or microporous), highloft nonwovens, polyolefin, polystyrene, polyurethane foams or particles, structures comprising a multiplicity of vertically oriented looped strands of fibers, absorbent core structures described above having punched holes or depressions, and the like. (As used herein, the term "microporous" refers to materials which are capable of transporting fluids by capillary action. The term "macroporous" refers to materials having pores too large to effect capillary transport of fluid, generally having pores greater than about 0.5 mm in diameter and, more specifically, having pores greater than about 1.0 mm in diameter.) One embodiment of a sublayer includes a mechanical fastening loop landing element, having an uncompressed thickness of about 1.5 millimeters available as XPL-7124 from the 3M Corporation of Minneapolis, Minnesota. Other suitable absorbent and nonabsorbent sublayers are described in U.S. Patent No. 6,680,422 entitled "Disposable Absorbent Article Having Capacity to Store Low-Viscosity Fecal Material" issued to Roe on January 20, 2004 and U.S. Patent No. 5,941,864 entitled "Disposable Absorbent Article Having Improved Fecal Storage" issued to Roe on August 24, 1999. Further, the sublayer, or any portion thereof, may include or be coated with a lotion or other known substances to add, enhance or change the performance or other characteristics of the element.

The diaper 720 may also comprise at least one elastic waist feature 734 that helps to provide improved fit and containment. The elastic waist feature 734 is generally intended to elastically expand and contract to dynamically fit the wearer's waist. The elastic waist feature 734 preferably extends at least longitudinally outwardly from at least one waist edge 762 of the absorbent core 728 and generally forms at least a portion of the end edge 752 of the diaper 720. Disposable diapers are often constructed so as to have two elastic waist features, one positioned in the first waist region 736 and one positioned in the second waist region 738. Further, while the elastic waist feature 734 or any of its constituent elements may comprise one or more separate elements affixed to the diaper 720, the elastic waist feature 734 may be constructed as an extension of other elements of the diaper 720, such as the backsheet 726, the topsheet 724, or both the backsheet 726 and the topsheet 724.

The elastic waist feature 734 may be constructed in a number of different configurations including those described in U.S. Pat. No. 4,515,595 issued to Kievit et al. on May 7, 1985; U.S. Pat. No. 4,710,189 issued to Lash on December 1, 1987; U.S. Pat. No. 5,151,092 issued to Buell on September 9, 1992; and U.S. Pat. No. 5,221,274 issued to Buell on June 22, 1993. Other waist configurations may include waistcap features such as described in U.S. Pat. No. 5,026,364 issued to Robertson on June 25, 1991 and U.S. Pat. No. 4,816,025 issued to Foreman on March 28, 1989.

The diaper 720 may also include a fastening system 740. The fastening system 740 preferably maintains the first waist region 736 and the second waist region 738 in a configuration so as to provide lateral tensions about the circumference of the diaper 720 to hold the diaper 720 on the wearer. The fastening system 740 preferably comprises a fastener such as tape tabs, hook and loop fastening components, interlocking fasteners such as tabs & slots, buckles, buttons, snaps, and/or hermaphroditic fastening components, although any other known fastening means are generally acceptable. Some exemplary surface fastening systems are disclosed in U.S. Patent 3,848,594 to Buell on November 19, 1974; U.S. Patent B1 4,662,875 to Hirotsu et al. on May 5, 1987; U.S. Patent 4,846,815 to Scripps on July 11, 1989; U.S. Patent 4,894,060 to Nestegard on January 16, 1990; U.S. Patent 4,946,527 to Battrell on August 7, 1990; the herein before referenced U.S. Pat. No. 5,151,092 issued to Buell on September 9, 1992; and U.S. Pat. No. 5,221,274 issued to Buell on June 22, 1993. An exemplary interlocking fastening system is disclosed in U.S. Patent No. 6,432,098 entitled "Absorbent Article Fastening Device" in the names of Kline et al. issued on August 13, 2002. The fastening system 740 may also provide a means for holding the article in a disposal configuration as disclosed in U.S. Pat. No. 4,963,140 issued to Robertson et al. on October 16, 1990. The fastening system 740 may also include primary and secondary fastening systems, as disclosed in U.S. Pat. No. 4,699,622 entitled "Disposable Diaper Having An Improved Side Closure" issued to Toussant et al. on October 13, 1987. The fastening system 740 may also reduce shifting of overlapped portions or improve fit as disclosed in U.S. Pat. No. 5,242,436 entitled "Absorbent Article With Fastening System Providing Dynamic Elasticized Waistband Fit" issued to Weil et al. on September 7, 1993; U.S. Pat. No. 5,499,978 entitled "Absorbent Article With Dynamic Elastic Waist Feature Having A Predisposed Resilient Flexural Hinge" issued to Buell et al. on March 19, 1996; U.S. Pat. No. 5,507,736 entitled "Absorbent Article With Dynamic Elastic Waist Feature Comprising An Expansive Tummy Panel" issued to Clear et al. on April 16, 1996; U.S. Pat. No. 5,591,152 entitled "Absorbent Article With Dynamic Elastic Waist Feature Having A Predisposed Resilient Flexural Hinge" issued to Buell et al. on January 7, 1997.

In alternative embodiments, the article may be pre-formed by the manufacturer to create a pant. The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be pre-formed by any suitable technique including, but not limited to, joining together portions of the article using refastenable and/or non-refastenable bonds (e.g., seam, weld, adhesive, cohesive bond, fastener, etc.). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13,2002; U.S. Patent No. 5,897,545, issued to Kline et al. on April 27, 1999; U.S. Patent No. 5,957,908, issued to Kline et al on September 28, 1999.

The diaper 720 may also comprise side panels 730. The side panels 730 may be elastic or extensible to provide a more comfortable and contouring fit by initially conformably fitting the diaper 720 to the wearer and sustaining this fit throughout the time of wear well past when the diaper 720 has been loaded with exudates since the elasticized side panels 730 allow the sides of the diaper 720 to expand and contract. The side panels 730 may also provide more effective application of the diaper 720 because even if the diaperer pulls one elasticized side panel 730 farther than the other during application, the diaper 720 will "self-adjust" during wear.

The diaper 720 may be provided with side panels 730 disposed in the first waist region 736, in the second waist region 738, or in both the first waist region 736 and the second waist region 738. Exemplary construction and configuration of side panels 730 are disclosed in U.S. Patent 4,857,067, entitled "Disposable Diaper Having Shirred Ears" issued to Wood, et al. on August 15, 1989; U.S. Patent 4,381,781 issued to Sciaraffa, et al. on May 3, 1983; U.S. Patent 4,938,753 issued to Van Gompel, et al. on July 3, 1990; the herein before referenced U.S. Pat. No. 5,151,092 issued to Buell; U.S. Pat. No. 5, 221,274 issued to Buell; U.S. Patent No. 5,669,897 issued to LaVon, et al. on September 23, 1997 entitled "Absorbent Articles Providing Sustained Dynamic Fit"; and U.S. Patent No. 6,004,306 entitled "Absorbent Article With Multi-Directional Extensible Side Panels" issued to Robles et al. on December 21, 1999.

The diaper 720 preferably further includes leg cuffs 732 which provide improved containment of liquids and other body exudates. Leg cuffs 732 may also be referred to as leg bands, side flaps, barrier cuffs, or elastic cuffs. For example, the diaper may include one or more first cuffs which provide improved containment of liquids and other body exudates. First cuffs may also be referred to as outer leg cuff, leg bands, side flaps, leg cuffs or elastic cuffs. U.S. Pat. No. 3,860,003 describes a disposable diaper which provides a contractible leg opening having a side flap and one or more elastic members to provide an elasticized leg cuff. Additionally, the diaper may include one or more second cuffs which also provide improved containment of liquids and other body exudates. Second cuffs may also be referred to as barrier leg cuffs, inner leg cuffs or "stand-up" elasticized flaps. U.S. Patent Nos. 4,808,178 and 4,909,803 issued to Aziz et al. on February 28, 1989 and March 20, 1990, respectively, describe disposable diapers having "stand-up" elasticized flaps which improve the containment of the leg regions. U.S. Patents Nos. 4,695,278 and 4,795,454 issued to Lawson on September 22, 1987 and to Dragoo on January 3, 1989, respectively, describe disposable diapers having dual cuffs, including first cuff and second cuff.

In some embodiments, it may be desirable to treat all or a portion of the leg cuffs 732 with a lotion, as described above. The leg cuffs may further be constructed in a number of different configurations, including those described in US Patent Nos. 4,636,207; 4,704,115; 4,900,317; 5,085,654; 5,492,751; 6,476,288; and SIR H1630. Any of the leg cuffs disclosed herein as well as other absorbent article components may also be fully or partially coated with a hydrophobic surface coating as detailed in copending U.S. Application No. 11/055743, which was filed February 10,2005.

Some embodiments may also include pockets for receiving and containing waste, spacers which provide voids for waste, barriers for limiting the movement of waste in the article, compartments or voids which accept and contain waste materials deposited in the diaper 720, and the like, or any combinations thereof. Examples of pockets and spacers for use in absorbent products are described in U.S. Patent 5,514,121 issued to Roe et al. on May 7, 1996, entitled "Diaper Having Expulsive Spacer"; U.S. Patent 5,171,236 issued to Dreier et al. on December 15, 1992 entitled "Disposable Absorbent Article Having Core Spacers"; U.S. Patent 5,397,318 issued to Dreier on March 14, 1995 entitled "Absorbent Article Having A Pocket Cuff'; U.S. Patent 5,540,671 issued to Dreier on July 30, 1996 entitled "Absorbent Article Having A Pocket Cuff With An Apex"; U.S. Patent No. 6,168,584 entitled "Spacers For Use In Hygienic Absorbent Articles And Disposable Absorbent Articles Having Such Spacer" issued to Allen et al. on January 2, 2001; U.S. Patent 5,306,266 entitled "Flexible Spacers For Use In Disposable Absorbent Articles" issued to Freeland on April 26, 1994; and U.S. Patent 5,997,520 entitled "Disposable Absorbent Article With Selectively Expandable or Inflatable Component" issued to Ahr et al. on December 7, 1999. Examples of compartments or voids are disclosed in U.S. Patent 4,968,312 entitled "Disposable Fecal Compartmenting Diaper" issued to Khan on November 6, 1990; U.S. Patent 4,990,147 entitled "Absorbent Article With Elastic Liner For Waste Material Isolation" issued to Freeland on February 5, 1991; U.S. Patent 5,062,840, entitled "Disposable Diapers" issued to Holt et al on November 5, 1991; U.S. Patent 5,269,755 entitled "Trisection Topsheets For Disposable Absorbent Articles And Disposable Absorbent Articles Having Such Trisection Topsheets" issued to Freeland et al on December 14, 1993; and U.S. Pat. Application No. 10/764,939 entitled "Articles with Cuffs" and filed on January 26, 2004 to Joerg Mueller et al.. Examples of suitable transverse barriers are described in U.S. Pat. No. 5,554,142 entitled "Absorbent Article Having Multiple Effective Height Transverse Partition" issued September 10, 1996 in the name of Dreier et al.; PCT Patent WO 94/14395 entitled "Absorbent Article Having An Upstanding Transverse Partition" published July 7, 1994 in the name of Freeland, et al.; and U.S. Patent No. 5,653,703 Absorbent Article Having Angular Upstanding Transverse Partition issued Aug. 5, 1997 to Roe, et al. Examples of other structures especially suitable for management of low viscosity feces are disclosed in U.S. Patents 5,941,864 issued to Roe et al. on August 24, 1999; U.S. Patent No. 5,977,430 issued to Roe et al. on Nov. 2, 1999 and 6,013,063 issued to Roe et al. on January 11, 2000.

The nonwoven laminate of the present invention may be used within a multitude of diaper elements as described above. Preferrably, the nonwoven laminate of the present invention may be used in place of any of the nonwovens described in the diapers or diaper elements above. Furthermore, the nonwoven laminate of the present invention may be used in place of other substrates wherein the opacity and/or barrier characteristics of the nonwoven laminate of present invention are desired. In one embodiment, the nonwoven laminate of the present invention may be used as a leg cuff. The nonwoven laminate of the present invention provide barrier protection and breathability while the high opacity masks underlying waste. In one embodiment, the nonwovens of the present invention may be used as a barrier layer within an absorbent article. The nonwoven laminate may be used as a barrier layer such as a backsheet, topsheet, anal cuff, outer cover, and barrier cover.

Furthermore, the nonwoven laminate of the present invention may be use as a substrate for a wet wipe. Construction of a typical wet wipe is illustrated in U.S. Publication 2004/009431 A1 entitled "Process for Making a Wet Wipe Using a Concentrated Emulsion" to S. Chamba et al. and in U.S. Publication 2005/0008681 A1 entitled "Composition For Wet Wipes Enhancing the Efficacy of Cleansing While Being Gentle To The Skin" to G.E. Deckner et al.

Furthermore, the nonwoven laminate of the present invention may be used in nonabsorbent articles including but not limited to tents, car covers, industrial applications (e.g., geo-textiles), drapes, gowns, footcovers, and sterilization wraps.

Contrast Ratio Method

Opacity is a measure of the capacity of a material to obscure the background behind it. The necessary equipment for the measurement of opacity includes:

- spectrophotometer such as Hunter Labscan XE (Model No. D25DP9000, with 45°/0° optics set-up) available from HunterLab Associates, Inc., Reston, VA or an equivalent 45°/0° spectrophotometer,
- black and white standard plates/tiles for calibration and measurements (also available from HunterLab Associates, Inc., or another colorimeter company)
- any soft absorbent tissue without embossing or lotion such as Puffs® for cleaning the standard plates, and
- a cutter for sizing the samples (e.g., Alfa cutter, scissors, paper cutter).

Since normal laboratory temperature and humidity ranges have a negligible effect upon opacity, the samples need not be conditioned prior to analysis. However, the samples and instrument should be kept in an area free of high humidity and corrosive vapors. Furthermore, the samples should be protected from contamination by dirt or lint.

Samples are prepared by cutting approximately a 10.16 cm x 10.16 cm portion from the web or laminate to be analyzed. Most samples are easily cut using a cutting die with a hydraulic cutter such as an Alfa cutter. Scissors or a paper cutter may be used; however, care must be taken to ensure that cutting does not destroy product web if needed for other analyses. Single 1-ply samples approximately 10.16 cm x 10.16 cm are to cut from the web or laminate with the machine direction perpendicular and/or parallel to the cut edges. A sample is selected for testing so as to be free from creases, wrinkles, tears, and other obvious defects. The sample is positioned so that the outer surface of the product as it is converted will be the top surface of the sample directly under the instrument sample port. If sheet orientation exists, the samples are positioned so that the machine direction is identical for all samples.

The spectrophotometer is calibrated using black and white standard tiles supplied with the instrument according to the manufacturer's instructions or other accepted standards of practice before beginning the testing. The color scale is set to XYZ, the Observer is set to 10°, and the illuminant is set to D65.

The white standard plate and the sample are placed into or onto the spectrophotometer according to the manufacturer's instructions. The sample is placed between the spectrophotometer orifice and the white standard plate without contaminating the test area of the sample. Light pulses and measurements are made against the white tile. Then, the sample is placed between the orifice and the black standard tile, and light pulses and measurements are made. The resulting data is read from and processed by the spectrophotometer and the "Y" (opacity) value is recorded to the nearest 0.1 unit. This procedure is repeated for a total of three representative replicates of the sample. The three Y values are averaged to yield the "Y" value for the given sample.

Opacity is reported as a percentage and is calculated by dividing the reflectance obtained from the sample with a black plate by the reflectance obtained from the sample with a white plate which is then multiplied by 100; the equation is as follows:

$$\text{Percent Opacity} = \left[ \frac{\text{Y value with black plate}}{\text{Y value with white plate}} \right] \times 100$$

This method of calculating opacity is commonly called the "contrast ratio method."

The opacity is measured as a percentage. The opacity of the nonwoven laminate of the present invention generally will be several percentage points greater than the opacity nonwoven laminate containing continuous filaments of a single cross-sectional shape, traditionally circular. The laminates in the present invention have an opacity greater than comparable laminates without shaped fibers (e.g., comparative laminates with similar basis weights made with substantially circular cross-sectional shaped continuous filaments). Preferably, the laminate of the present invention have an opacity of about 10% greater than comparable laminates without differing shaped continuous filaments; more preferably an opacity about 20% greater than comparable laminates without differing shaped continuous filaments; and most preferably an opacity about 30% greater than comparable laminates without differing shaped continuous filaments.

Opacity may be measured according to TAPPI Test Method T 426 om-01 with resulting opacity data being envisioned as approximately proportional to the data presented according to the method described above.

Basis weight is the mass per unit area of the substrate. Independent measurements of the mass and area of a specimen substrate may be taken and used to calculate the ratio of mass per unit area.

Examples

All the examples below may be analyzed as laminates prior to inclusion with an absorbent article or as a particular component within an absorbent article. However, it is envisioned that the laminates provided below may form at least a portion of an absorbent article, which are described more in greater detail above.

Example 1 is a trilobal spunbond + spunbond/meltblown/meltblown/spunbond (S+SMMS) laminate of the present invention. The shaped S-layer is a spunbonded polypropylene web of a mixture of filaments with differing cross-sectional shapes. The web comprises approximately a 50/50 mixture, by weight, of trilobal shaped filaments and substantially circular shaped filaments. The basis weight of the shaped S-layer is 8 gsm, and the approximate filament denier (grams/9,000 meters) is 1.25. The web is available from Hill, Inc., West Melbourne, FL. The trilobal shaped S-layer is substantially free of colorants. The SMS layer comprises two meltblown layers with a basis weight of 1 gsm each between two spunbond layer with a basis weight of 4 gsm each. Within the SMMS layer, the M and/or S webs may further contain colorants (e.g., dyes and pigments such as $TiO_2$) up to about 0.5%, by weight, if present. The S and SMMS layers are not bound to one other.

Example 2 is a comparative circular spunbond + spunbond/meltblown/meltblown/spunbond (S+SMMS) laminate. The shaped S-layer is a spunbonded polypropylene web having a basis weight of 6 gsm and an approximate denier (grams/9,000 meters) of 1.5. The cross-sectional shape of the filaments is substantially circular. The substantially circular shaped S-layer web is available from Nordson Corp., Westlake, OH. The substantially circular shaped S-layer may contain colorants (e.g., $TiO_2$) up to about 0.5%, by weight, if present. The SMMS layer is that of Example 1. The S and SMMS layers are not bound to one other.

Example 3 is a comparative circular spunbond + spunbond/meltblown/meltblown/spunbond (S+SMMS) laminate. The shaped S layer is a spunbonded polypropylene web having a basis weight of 10 gsm and an approximate denier (grams/9,000 meters) of 2. The cross-sectional shape of the filaments is substantially circular. The substantially circular shaped S-layer web is available from Avgol Nonwoven Industries, Isreal. The substantially circular shaped S-layer may contain colorants (e.g., $TiO_2$) up to about 0.5%, by weight, if present. The SMS layer is that of Example 1. The S and SMMS layers are not bound to one other.

| Example | Total Basis Weight (gsm) | Opacity (%) |
|---------|--------------------------|-------------|
| 1 | 18 | 37.9 |
| 2 | 16 | 29.0 |
| 3 | 20 | 35.8 |

Example 4 is a trilobal spunbond + spunbond/meltblown/meltblown/spunbond (S+SMMS) laminate of the present invention. The shaped S-layer is a spunbonded polypropylene web of a mixture of filaments with differing cross-sectional shapes. The web comprises approximately a 50/50 mixture, by weight, of trilobal shaped filaments and substantially circular shaped filaments. The basis weight of the shaped S-layer is 16 gsm, and the approximate filament denier (grams/9,000 meters) is 1.25. The web is available from Hill, Inc., West Melbourne, FL. The trilobal shaped S-layer is substantially free of colorants. The SMMS layer is that of Example 1. The S and SMMS layers are not bound to one other.

Example 5 is a comparative circular spunbond/meltblown/meltblown/spunbond (S+SMMS) laminate. The shaped S layer is a spunbonded polypropylene web having a basis weight of 16 gsm and approximate denier (grams/9,000 meters) of 2-2.5. The shaped S-layer was a spunbonded polypropylene web of filaments having a substantially circular cross-sectional shape. The web is available from BBA Nonwovens, Old Hickory, TN. The substantially circular shaped S-layer may contain colorants (e.g., $TiO_2$) up to about 0.5%, by weight, if present. The SMMS layer is that of Example 1. The S and SMMS layers are not bound to one other.

| Example | Total Basis Weight (gsm) | Opacity (%) |
|---------|--------------------------|-------------|
| 4 | 26 | 47.5 |
| 5 | 26 | 38.6 |

Example 6 is a trilobal spunbond + spunbond/meltblown/meltblown/spunbond (S+SMMS) laminate of the present invention. The shaped S-layer is a spunbonded polypropylene web of trilobal shaped filaments. The basis weight of the shaped S-layer is 8 gsm, and the approximate filament denier (grams/9,000 meters) is 1.25. The web is available from Hill, Inc., West Melbourne, FL. The trilobal shaped S-layer is substantially free of colorants. The SMMS layer is that of Example 1. The S and SMMS layers are not bound to one other.

Example 7 is the S+SMMS laminate of Example 1 (or any of the nonwoven laminates of the present invention) within the disposable absorbent article as disclosed in co-assigned U.S. Patent No. 5,085,654 to Buell. Within the disclosure, the cuff (e.g., callout 15 in Buell) may comprise, in part, the S+SMS laminate. In particular, the S+SMS laminate serves as breathable element (e.g., callout 54 in Buell) of cuff as presented in Fig. 5 of U.S. Patent No. 5,085,654.

## Claims

1. An absorbent article comprising a nonwoven laminate comprising:

    a) a first nonwoven layer comprising first continuous filaments;
    b) a second nonwoven layer comprising second continuous filaments;
    c) a third nonwoven layer comprising fine fibers, said second nonwoven layer between said first and third nonwoven layers; and
    d) an optional fourth nonwoven layer comprising second continuous filaments;

    said nonwoven laminate is **characterized in that**

    1) said first and said second continuous filaments have cross-sectional shapes that are distinct from one another;
    2) said second nonwoven layer and said optional fourth nonwoven layer each have a basis weight of less than 5 g/m$^2$, preferably less than 4 g/m$^2$; and
    3) the total basis weight of the first, second, and third layers is not greater than about 55 g/m$^2$; and
    4) said cross-sectional shape of the first continuous filaments is trilobal.

2. The absorbent article of Claim 1 wherein the first nonwoven layer has a basis weight from about 4 g/m$^2$ to about 7 g/m$^2$ and the third nonwoven layer has a basis weight from about 0.5 g/m$^2$ to about 5 g/m$^2$.

3. The absorbent article of any of the preceding claims wherein the nonwoven laminate has an opacity greater than a similarly constructed nonwoven, said similarly constructed nonwoven having continuous filaments with substantially circular cross-sectional shapes.

4. The absorbent article of any of the preceding claims further comprising a leg cuff comprised of the nonwoven laminate.

5. The absorbent article of Claim 1 wherein the first nonwoven layer comprising at least two first continuous filaments having distinct cross-sectional shapes, wherein at least 5%, by weight, of first continuous filaments are different in cross-sectional shape than the cross-sectional shape of the second continuous filaments in the second nonwoven layer.

6. The absorbent article of Claim 5 wherein the first nonwoven layer has a basis weight of about 4 g/m$^2$ to about 7 g/m$^2$ and the third nonwoven layer has a basis weight of about 0.5 g/m$^2$ to about 5 g/m$^2$.

**Patentansprüche**

1. Absorptionsartikel, umfassend ein Vlieslaminat, umfassend:

a) eine erste Vliesschicht, umfassend erste Endlosfäden,
b) eine zweite Vliesschicht, umfassend zweite Endlosfäden,
c) eine dritte Vliesschicht, umfassend feine Fasern, wobei die zweite Vliesschicht zwischen der ersten und der dritten Vliesschicht angeordnet ist, und
d) eine fakultative vierte Vliesschicht, umfassend zweite Endlosfäden, wobei das Vlieslaminat **dadurch gekennzeichnet ist, dass**

1) die ersten und die zweiten Endlosfäden Querschnittsformen aufweisen, die sich voneinander unterscheiden,
2) die zweite Vliesschicht und die fakultative vierte Vliesschicht jeweils ein Flächengewicht von weniger als 5 g/m$^2$, vorzugsweise weniger als 4 g/m$^2$ aufweisen, und
3) das gesamte Flächengewicht der ersten, zweiten und dritten Schicht nicht größer als ungefähr 55 g/m$^2$ ist und
4) die Querschnittsform der ersten Endlosfäden trilobal ist.

2. Absorptionsartikel nach Anspruch 1, wobei die erste Vliesschicht ein Flächengewicht von ungefähr 4 g/m$^2$ bis ungefähr 7 g/m$^2$ aufweist und die dritte Vliesschicht ein Flächengewicht von ungefähr 0,5 g/m$^2$ bis ungefähr 5 g/m$^2$ aufweist.

3. Absorptionsartikel nach einem der vorstehenden Ansprüche, wobei das Vlieslaminat eine größere Trübung als ein ähnlich aufgebauter Vliesstoff aufweist, wobei der ähnlich aufgebaute Vliesstoff Endlosfäden mit im Wesentlichen kreisförmigen Querschnittsformen aufweist.

4. Absorptionsartikel nach einem der vorstehenden Ansprüche, der ferner ein Beinbündchen umfasst, das aus dem Vlieslaminat besteht.

5. Absorptionsartikel nach Anspruch 1, wobei die erste Vliesschicht mindestens zwei erste Endlosfäden mit unterschiedlichen Querschnittsformen umfasst, wobei mindestens 5 Gew.-% der ersten Endlosfäden eine andere Querschnittsform aufweisen als die Querschnittsform der zweiten Endlosfäden in der zweiten Vliesschicht.

6. Absorptionsartikel nach Anspruch 5, wobei die erste Vliesschicht ein Flächengewicht von ungefähr 4 g/m$^2$ bis ungefähr 7 g/m$^2$ aufweist und die dritte Vliesschicht ein Flächengewicht von ungefähr 0,5 g/m$^2$ bis ungefähr 5 g/m$^2$ aufweist.

**Revendications**

1. Article absorbant comprenant un stratifié non-tissé comprenant :

a) une première couche de non-tissé comprenant des premiers filaments continus ;

b) une deuxième couche de non-tissé comprenant des seconds filaments continus ;

c) une troisième couche de non-tissé comprenant des fibres fines, ladite deuxième couche de non-tissé étant située entre lesdites première et troisième couches de non-tissé ; et

d) une quatrième couche de non-tissé en option comprenant des seconds filaments continus ; ledit stratifié non-tissé est **caractérisé en ce que**

1) lesdits premier et second filaments continus ont des formes en coupe transversale qui diffèrent l'une de l'autre ;

2) ladite deuxième couche de non-tissé et ladite quatrième couche de non-tissé en option ont chacune un poids de base inférieur à 5 g/m$^2$, de préférence inférieur à 4 g/m$^2$ ; et

3) le poids de base total des première, deuxième, et troisième couches n'est pas supérieur à environ 55 g/m$^2$ ; et

4) ladite forme en coupe transversale des premiers filaments continus est trilobée.

2. Article absorbant selon la revendication 1, dans lequel la première couche de non-tissé a un poids de base d'environ 4 g/m$^2$ à environ 7 g/m$^2$, et la troisième couche de non-tissé a un poids de base d'environ 0,5 g/m$^2$ à environ 5 g/m$^2$.

3. Article absorbant selon l'une quelconque des revendications précédentes, dans lequel le stratifié non-tissé présente une opacité supérieure à un non-tissé construit de manière similaire, ledit non-tissé construit de manière similaire ayant des filaments continus avec des formes en coupe transversale sensiblement circulaires.

4. Article absorbant selon l'une quelconque des revendications précédentes, comprenant en outre une collerette de jambe constituée du stratifié non-tissé.

5. Article absorbant selon la revendication 1, dans lequel la couche de non-tissé comprend au moins deux premiers filaments continus ayant des formes en coupe transversale différentes, dans lequel au moins 5 % en poids des premiers filaments continus sont différents en forme en coupe transversale de la forme en coupe transversale des seconds filaments continus dans la deuxième couche de non-tissé.

6. Article absorbant selon la revendication 5, dans lequel la première couche de non-tissé a un poids de base d'environ 4 g/m$^2$ à environ 7 g/m$^2$ et la troisième couche de non-tissé a un poids de base d'environ 0,5 g/m$^2$ à environ 5 g/m$^2$.

Fig. 1

Fig. 2

Fig. 3

Fig. 4

Fig. 5

Fig. 6a

Fig. 6b

Fig. 6c

Fig. 6d

Fig. 6e

Fig. 6f

Fig. 6g

Fig. 6h

Fig. 6i

Fig. 6j

Fig. 6k

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4900317 A
- US 73627104 A
- US 04734605 A
- US 3849241 A, Buntin
- US 4041203 A, Brock
- US 4374888 A
- US 5628097 A
- US 5916661 A
- US 909486 A
- US 3860003 A
- US 5151092 A, Buell
- US 5221274 A, Buell
- US 5554145 A
- US 5569234 A
- US 5580411 A
- US 6004306 A
- WO 9516746 A, E. I. DuPont
- US 5938648 A, LaVon
- US 5865823 A, Curro
- US 5571096 A, Dobrin
- US 5518801 A
- US 4573986 A
- US 3911173 A, Sprague, Jr.
- US 4785996 A, Ziecker
- US 4842666 A, Werenicz
- US 3929135 A, Thompson
- US 4324246 A, Mullane
- US 4342314 A, Radel
- US 4463045 A, Ahr
- US 5006394 A, Baird
- US 4609518 A
- US 4629643 A, Curro
- US 20040092902 A1, Anja Hoffmann
- US 4988344 A
- US 4988345 A
- US H1670 A, Aziz
- US 5607760 A, Roe
- US 5609587 A, Roe
- US 5635191 A, Roe
- US 5643588 A, Roe
- US 5968025 A, Roe
- WO 9524173 A
- US 4892536 A, DesMarais
- US 4990147 A, Freeland
- US 5037416 A, Allen
- US 5269775 A, Freeland
- US 6482191 B, Roe
- US 76485004 A
- US 703239 A
- US 20040092900 A1
- US 4610678 A
- US 4673402 A
- US 4834735 A
- US 4888231 A
- US 5137537 A
- US 5147345 A
- US 5342338 A
- US 5260345 A
- US 5387207 A
- US 5625222 A
- US 6680422 B
- US 5941864 A
- US 4515595 A, Kievit
- US 4710189 A, Lash
- US 5026364 A, Robertson
- US 4816025 A, Foreman
- US 3848594 A, Buell
- US 4662875 B1, Hirotsu
- US 4846815 A, Scripps
- US 4894060 A, Nestegard
- US 4946527 A, Battrell
- US 6432098 B
- US 4963140 A, Robertson
- US 4699622 A
- US 5242436 A
- US 5499978 A
- US 5507736 A
- US 5591152 A
- US 5246433 A, Hasse
- US 6120487 A, Ashton
- US 6120489 A, Johnson
- US 4940464 A, Van Gompel
- US 5092861 A, Nomura
- US 17124902 A
- US 5897545 A, Kline
- US 5957908 A, Kline
- US 4857067 A
- US 4381781 A, Sciaraffa
- US 4938753 A, Van Gompel
- US 5669897 A, LaVon
- US 4808178 A
- US 4909803 A
- US 4695278 A
- US 4795454 A
- US 4636207 A
- US 4704115 A
- US 5085654 A
- US 5492751 A

- US 6476288 A
- US 05574305 A
- US 5514121 A, Roe
- US 5171236 A, Dreier
- US 5397318 A, Dreier
- US 5540671 A, Dreier
- US 6168584 B
- US 5306266 A
- US 5997520 A
- US 4968312 A

- US 5062840 A
- US 5269755 A
- US 76493904 A
- US 5554142 A
- WO 9414395 A
- US 5653703 A
- US 5977430 A, Roe
- US 6013063 A, Roe
- US 2004009431 A1
- US 20050008681 A1

**Non-patent literature cited in the description**

- Manufacture of Super-Fine Organic Fibers'', by V. A. Wendt, E. L. Boon, and C. D. Fluharty. *NRL Report 4364*

- **K. D. LAWRENCE ; R. T. LUKAS ; J. A. YOUNG.** An Improved Device for the Formation of Super-Fine Thermoplastic Fibers. *NRL Report 5265*